# FASCICULE DE BREVET EUROPEEN

(11) **EP 1 439 791 B1**
(45) Date de publication et mention de la délivrance du brevet: **19.01.2005**
(21) Numéro de dépôt: 02790502.5
(22) Date de dépôt: 16.09.2002
(51) Int. Cl.: A61B 17/80, A61B 17/66

(54) **IMPLANT-PLAQUE**
PLATTENIMPLANTAT
PLATE-IMPLANT

(30) Priorité: 14.09.2001 FR 0111945
(43) Date de publication de la demande: 28.07.2004
(73) Titulaire: OBL (Société Anonyme), 92320 Chatillon (FR)
(72) Inventeur: LABBE, Daniel, F-14000 Caen (FR); SABIN, Pierre, F-76000 Rouen (FR)
(74) Mandataire: Degret, Jacques
(86) Numéro de dépôt international: PCT/FR2002/003155
(87) Numéro de publication internationale: WO 2003/024347

(56) Documents cités:
- FR-A- 2 738 477
- FR-A- 2 801 779
- US-A- 4 379 694
- US-A- 5 201 736

## Description

De nos jours, la chirurgie réparatrice du visage fait appel conjointement aux techniques de reconstruction osseuse issues de la méthode mise au point par le chirurgien russe Ilizarov, et à des prothèses faciales (oreille, nez, joue ...) fixées sur le substrat osseux.

La demande de brevet européen EP 0.433.852 déposée par la société Oswald Leibinger, publiée le 26 juin 1991, décrit un système de plaques d'implantologie destinées à fixer de telles prothèses. Le système commercialisé sous la marque EPITEC® consiste en une grille pouvant épouser une forme en trois dimensions quelconque, grille qui est ancrée au moyen de vis courtes dans des zones où une quantité suffisante d'os est disponible. La plaque comporte des inserts vissés dans lesquels sont placés des piliers supportant la prothèse.

Ce système permet une mise en place idéale de la prothèse même si l'os sous-jacent est défectueux, et ce grâce à la possibilité de déport de la zone de fixation dans une zone saine.

Cependant, ce type de plaque se révèle compliqué à utiliser, notamment parce qu'il faut la découper et la conformer en fonction des besoins pendant l'opération.

Dans certains cas, on préférera donc utiliser des moyens de fixation de la prothèse de type "implant endo-osseux", similaires aux implants dentaires.

Le brevet américain US 5.102.414 publié le 7 avril 1992 au nom de A. Kirsch décrit un implant extra-oral de ce type. La prothèse est fixée au moyen d'un pilier inséré dans un alésage axial de l'implant.

L'inconvénient de ce moyen d'ancrage est qu'il ne procure pas une stabilité rotationnelle suffisante à la prothèse : le corps de l'implant peut tourner dans le substrat osseux, alors même que le pilier est solidaire en rotation de l'implant, si celui-ci présente, par exemple, une tête hexagonale.

Une forme spéciale du filetage de l'implant peut favoriser l'ostéointégration, ainsi que le décrit la demande de brevet français FR 2.788.215, au nom de P. Sabin et autres, publiée le 13 juillet 2000. De ce fait, la liaison entre l'implant et l'os dans lequel il est ancré est plus fiable et plus durable, mais tout risque de rotation n'est pas écarté.

Un autre moyen pour augmenter la fiabilité de l'implantation en évitant les inconvénients du système EPITEC® est de recourir à des plaques en forme de croix, de patte-d'oie, ou similaire. Des plaques de ce type sont utilisées pour la fixation du distracteur mandibulaire intraoral décrit dans la demande de brevet français FR 2.801.779 (Le préamlule de la revendication 1 est basé sur ce document) publiée le 8 juin 2001 au nom de la société OBL. Les plaques, de forme générale allongée, présentent plusieurs branches comportant des perforations pour le passage des vis destinées à être implantées dans le corps mandibulaire. Sur l'une des deux faces de ce modèle de plaques, un bossage muni d'un trou cylindrique taraudé permet le vissage d'une vis d'articulation tout en évitant les frottements du bras du distracteur sur la plaque.

Les points d'ancrage multiples assurent une grande stabilité de la plaque, mais celle-ci n'est pas adaptée à la fixation des piliers percutanés couramment utilisés pour supporter les prothèses.

Une variante de plaque d'ostéosynthèse implantable en chirurgie maxillo-faciale à haut pouvoir d'accrochage est décrite dans la demande de brevet français FR 2.738.477 publiée le 14 mars 1997 au nom de Landanger Landos et autres, mais cette autre plaque d'ostéosynthèse ne permet pas davantage la fixation de piliers percutanés.

Il résulte donc de l'état de la technique détaillé ci-dessus que de nombreux types d'implants ou de plaques d'implantologie, tant extra-oraux qu'intra-oraux, sont connus, mais qu'il n'existe à ce jour aucun modèle d'implant-plaque répondant exactement aux besoins des spécialistes en chirurgie maxillo-faciale, à savoir assurer la stabilité rotationnelle soit de la prothèse ou d'un élément de fixation de ladite prothèse, soit encore d'un élément de distracteur.

### DESCRIPTION GÉNÉRALE DE L'INVENTION

La présente invention concerne donc un implant-plaque destiné à la fixation d'un pilier percutané, supportant une prothèse, ou d'une broche de distracteur et à garantir la stabilité rotationnelle d'une telle fixation. Selon un type de plaque connu en implantologie, cet implant-plaque présente plusieurs branches minces comportant des trous ou oeillets aptes à recevoir des vis d'ancrage dans un corps osseux. Selon une caractéristique également connue, les branches de l'implant-plaque sont réparties sensiblement dans un plan autour d'un bossage central présentant un alésage cylindrique taraudé d'axe perpendiculaire au plan de celle-ci.

L'invention a précisément pour objet un implant-plaque de ce type particulièrement adapté à l'implantologie ou à la distraction extra-orale, dont le bossage central présente dans ce but une partie extrémale de forme prismatique régulière d'axe longitudinal confondu avec l'axe de son alésage.

Un tel implant-plaque à plusieurs branches qui comportent chacune au moins un trou ou oeillet, et qui sont réparties dans un plan autour de son bossage central doté d'une telle particularité, convient par conséquent aux besoins thérapeutiques exigeant une implantation s'étendant sensiblement dans un plan sur la surface de l'os.

Fort avantageusement, cette forme prismatique est complémentaire de la forme de l'extrémité en regard du pilier supportant la prothèse, ou de l'extrémité de la broche du distracteur.

De préférence, cette forme prismatique extrémale du bossage est convexe, et elle présente une section droite hexagonale.

Selon un mode de réalisation avantageux de l'implant-plaque selon l'invention, celui-ci est en forme générale de T, le bossage central étant situé à la jonction de la barre et de la jambe du T.

La jambe du T comporte de préférence deux trous ou oeillets, tandis que chaque extrémité de la barre du T en comporte un seul.

Selon une autre caractéristique judicieuse de l'implant-plaque selon l'invention, le bossage central est un cylindre d'axe confondu avec l'axe de son alésage. Cet alésage est utilement borgne.

L'implant-plaque selon l'invention est réalisé en un matériau biocompatible, préférentiellement un alliage de titane.

Selon une forme préférée d'exécution, la partie extrémale du bossage central est de longueur sensiblement égale à 0,7 mm.

### BRÈVE DESCRIPTION DES DESSINS

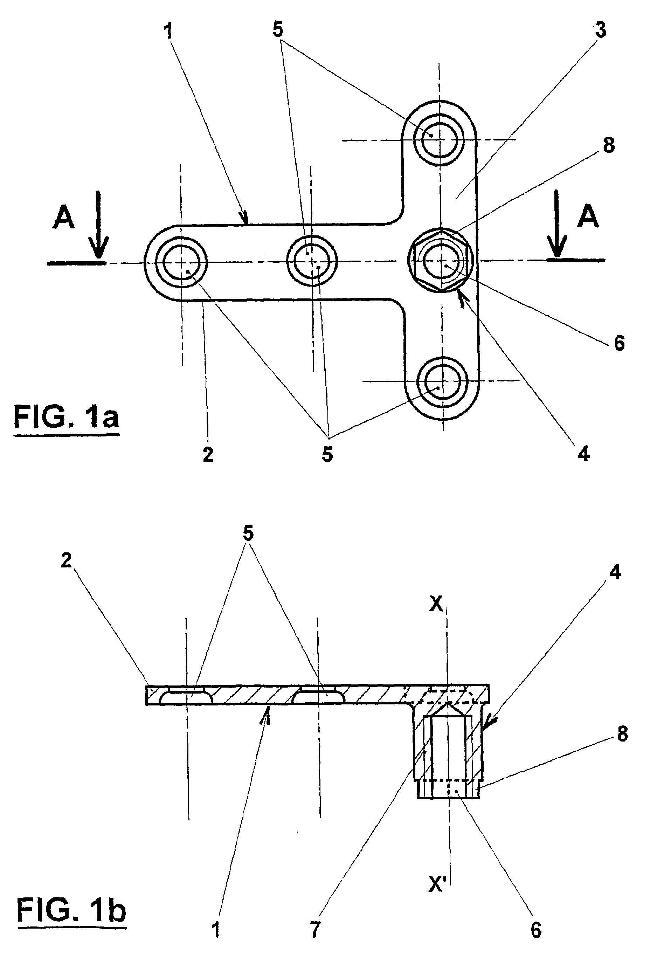

La Figure 1a est une vue de face d'une forme d'exécution préférée de l'implant-plaque selon l'invention.
La Figure 1b montre la coupe AA du même implant-plaque selon l'invention.

### DESCRIPTION D'UNE FORME D'EXÉCUTION PRÉFÉRÉE DE L'INVENTION

Les références aux Figures la et 1b serviront à expliquer en détail les caractéristiques d'un modèle d'implant-plaque 1 selon l'invention.

La plaque 1 est essentiellement formée d'une pièce en alliage de titane TA6V en forme générale de T à branches minces, d'une épaisseur de 8/10 de mm. Elle présente, au niveau de la jonction de la jambe 2 et de la barre 3 du T, une partie cylindrique 4, ou bossage central, d'axe XX' perpendiculaire à son plan, constituant une base destinée à fixer soit un pilier percutané supportant une prothèse, soit une broche de distracteur.

Les branches 2,3 de la plaque 1, c'est-à-dire la jambe 2 et la barre 3 du T, sont sensiblement coplanaires et elles comportent plusieurs trous ou oeillets 5 destinés à recevoir les vis d'ancrage dans le substrat osseux. Ces trous sont munis chacun d'un chanfrein sphérique afin d'améliorer la tenue des têtes des vis.

Le modèle de plaque plane 1 présenté comporte deux trous 5 dans la branche longitudinale ou jambe 2 et un trou 5 à chaque extrémité de la branche transversale ou barre 3. Cette plaque 1 mesure environ 13 mm X 13 mm. La largeur des branches 2,3 est de 4 mm. Les trous 5 d'ancrage ont 2 mm de diamètre. Il s'agit de dimensions courantes pour ce type de plaque, le but de l'invention étant notamment d'optimiser les caractéristiques des plaques d'implantologie utilisées en chirurgie maxillo-faciale, tout en restant compatible avec les matériels standards.

La partie cylindrique 4 centrale, de 3,5 mm de diamètre et d'environ 4 mm de hauteur, présente un alésage axial 6 comportant un filetage intérieur 7 au standard ISO M2, c'est-à-dire permettant de visser des piliers percutanés courants. Les axes de l'alésage 6 et respectivement de la partie cylindrique 4 sont donc confondus.

Cette partie 4, constituée d'un alliage de titane très résistant, est soudée au laser sur les branches 2,3 faites d'un alliage plus malléable.

La rotation du pilier par rapport à la plaque 1 est bloquée au moyen d'un surplat hexagonal extérieur 8 dont est munie la base 4 à son extrémité libre. La forme hexagonale du surplat 8 est à cet effet exactement complémentaire de celle du six-pans creux que comportent à leur extrémité les piliers standards. Sa hauteur est de 0,7 mm.

La coupe AA de la Figure 1b montre que l'alésage axial 6 de la base 4 est borgne. Il s'agit d'une caractéristique préférentielle mais, dans certains modèles d'implant-plaque, le trou 6 peut être débouchant.

Comme il va de soi, l'invention ne se limite pas aux seules spécifications techniques ci-dessus données à titre d'exemple; elle embrasse, au contraire, toutes les variantes possibles de réalisation.

En particulier, la forme générale en T de la plaque décrite n'est pas limitative. L'homme de l'art connaît la diversité des exécutions possibles, et tout autre modèle analogue d'implant-plaque permettant que cet implant soit fixé sur la surface de l'os et présentant les caractéristiques spécifiées ci-après ne sortirait pas du concept inventif d'implant-plaque décrit.

## Revendications

1. Implant-plaque (1) destiné à la fixation d'un pilier percutané supportant une prothèse ou d'une broche de distracteur, ledit implant-plaque (1) étant du type de ceux présentant plusieurs branches (2,3) minces comportant des trous ou oeillets (5) aptes à recevoir des vis d'ancrage dans un corps osseux, et lesdites branches (2,3) étant réparties sensiblement dans un plan autour d'un bossage central (4) présentant un alésage cylindrique taraudé (6) d'axe (XX') perpendiculaire au plan dudit implant-plaque (1), **caractérisé en ce que** ledit bossage (4) présente une partie extrémale (8) de forme prismatique régulière d'axe longitudinal (XX') confondu avec l'axe (XX') dudit alésage (6).

2. Implant-plaque (1) selon la revendication 1, **caractérisé en ce que** la forme prismatique de la partie extrémale (8) du bossage (4) est complémentaire de la forme de l'extrémité en regard dudit pilier ou de ladite broche.

3. Implant-plaque (1) selon l'une quelconque des revendications 1 ou 2, **caractérisé en ce que** la forme prismatique de la partie extrémale (8) est convexe.

4. Implant-plaque (1) selon l'une quelconque des revendications 1 à 3, **caractérisé en ce que** la forme prismatique de la partie extrémale (8) présente une section droite hexagonale.

5. Implant-plaque (1) selon l'une quelconque des revendications 1 à 4, **caractérisé en ce qu'**il est en forme générale de T, le bossage (4) étant situé à la jonction de la barre (3) et de la jambe (2) dudit T.

6. Implant-plaque (1) selon la revendication 5, **caractérisé en ce que** ladite jambe (2) du T comporte deux desdits trous ou oeillets (5) et **en ce que** chaque extrémité de ladite barre (3) du T comporte un desdits trous ou oeillets (5).

7. Implant-plaque (1) selon l'une quelconque des revendications 1 à 6, **caractérisé en ce que** le bossage (4) est un cylindre (4) d'axe (XX') confondu avec l'axe (XX') de l'alésage (6).

8. Implant-plaque (1) selon l'une quelconque des revendications 1 à 7, **caractérisé en ce que** ledit alésage (6) est borgne.

9. Implant-plaque (1) selon l'une quelconque des revendications 1 à 8, **caractérisé en ce qu'**il est réalisé en un matériau biocompatible, préférentiellement un alliage de titane.

10. Implant-plaque (1) selon l'une quelconque des revendications 1 à 9, **caractérisé en ce que** ladite partie extrémale (8) est préférentiellement de longueur sensiblement égale à 0,7 mm.

## Patentansprüche

1. Plattenimplantat (1), das zur Befestigung einer perkutanen Stütze, die eine Prothese trägt, oder eines Distraktorstiftes dient, wobei das Plattenimplantat (1) von jenem Typ ist, der mehrere dünne Arme (2, 3) aufweist, die Löcher oder Ösen (5) aufweisen, die in der Lage sind Ankerschrauben in einem Knochenkörper aufzunehmen, und die Arme (2, 3) im wesentlichen in einer Ebene um einen zentralen Vorsprung (4) verteilt angeordnet sind, der eine zylindrische Gewindeausnehmung (6) mit einer Achse (XX') aufweist, die senkrecht zur Ebene des Plattenimplantats (1) verläuft, **dadurch gekennzeichnet, dass** der Vorsprung (4) einen Endabschnitt (8) von regelmäßiger prismatischer Form mit Längsachse (XX') aufweist, die mit der Achse (XX') der Ausnehmung (6) zusammenfällt.

2. Plattenimplantat (1) nach Anspruch 1, **dadurch gekennzeichnet, dass** die prismatische Form des Endabschnitts (8) des Vorsprungs (4) komplementär ist bezüglich der Form des Endes der Stütze oder des Stiftes.

3. Plattenimplantat (1) nach Anspruch 1 oder 2, **dadurch gekennzeichnet, dass** die prismatische Form des Endabschnitts (8) konvex ist.

4. Plattenimplantat (1) nach einem der Ansprüche 1 bis 3, **dadurch gekennzeichnet, dass** die prismatische Form des Endabschnitts (8) einen hexagonalen Querschnitt aufweist.

5. Plattenimplantat (1) nach einem der Ansprüche 1 bis 4, **dadurch gekennzeichnet, dass** es allgemein die Form eines T aufweist, wobei der Vorsprung (4) an der Verbindungsstelle des Balkens (3) mit dem Schenkel (2) des T angeordnet ist.

6. Plattenimplantat (1) nach Anspruch 5, **dadurch gekennzeichnet, dass** der Schenkel (2) des T zwei der Löcher oder Ösen (5) aufweist, und dass jedes Ende des Balkens (3) des T eines der Löcher bzw. eine der Ösen (5) aufweist.

7. Plattenimplantat (1) nach einem der Ansprüche 1 bis 6, **dadurch gekennzeichnet, dass** der Vorsprung (4) ein Zylinder (4) mit einer Achse (XX') ist, die mit der Achse (XX') der Ausnehmung (6) zusammenfällt.

8. Plattenimplantat (1) nach einem der Ansprüche 1 bis 7, **dadurch gekennzeichnet, dass** die Ausnehmung (6) ein Blindloch ist.

9. Plattenimplantat (1) nach einem der Ansprüche 1 bis 8, **dadurch gekennzeichnet, dass** es aus einem biokompatiblen Material gebildet ist, vorzugsweise einer TitanLegierung.

10. Plattenimplantat (1) nach einem der Ansprüche 1 bis 9, **dadurch gekennzeichnet, dass** der Endabschnitt (8) vorzugsweise von einer Länge im wesentlichen gleich 0,7 mm ist.

## Claims

1. Plate implant (1) intended for fixing a percutaneous pillar supporting a prosthesis or a distractor pin, said plate implant (1) being of the type comprising a plurality of thin branches (2, 3) having holes or eyelets (4) adapted to accommodate screws for anchoring in a bony structure, and said branches (2, 3) being distributed substantially in a plane around a central boss (4) having a screw-threaded cylindrical bore (6) the axis (XX') of which is perpendicular to the plane of said plate implant (1), **characterised in that** the boss (4) has an end portion (8) of a regular prismatic shape the longitudinal axis (XX') of which coincides with the axis (XX') of said bore (6).

2. Plate implant (1) according to claim 1, **characterised in that** the prismatic shape of the end portion (8) of the boss (4) is complementary to the shape of the facing end of said pillar or said pin.

3. Plate implant (1) according to any one of claims 1 and 2, **characterised in that** the prismatic shape of the end portion (8) is convex.

4. Plate implant (1) according to any one of claims 1 to 3, **characterised in that** the prismatic shape of the end portion (8) is hexagonal in section.

5. Plate implant (1) according to any one of claims 1 to 4, **characterised in that** it is generally T-shaped, the boss (4) being located at the junction of the crosspiece (3) and upright (2) of said T.

6. Plate implant (1) according to claim 5, **characterised in that** the upright (2) of the T comprises two of said holes or eyelets (5) and **in that** each end of said crosspiece (3) of the T comprises one of said holes or eyelets (5).

7. Plate implant (1) according to any one of claims 1 to 6, **characterised in that** the boss (4) is a cylinder (4) the axis (XX') of which coincides with the axis (XX') of the bore (6).

8. Plate implant (1) according to any one of claims 1 to 7, **characterised in that** the bore (6) is a blind bore.

9. Plate implant (1) according to any one of claims 1 to 8, **characterised in that** it is made of a biocompatible material, preferably a titanium alloy.

10. Plate implant (1) according to any one of claims 1 to 9, **characterised in that** the end portion (8) preferably has a length substantially equal to 0.7 mm.
